(19) Europäisches Patentamt
European Patent Office
Office européen des brevets

(11) Publication number: **0 063 105**
**B1**

(12) **EUROPEAN PATENT SPECIFICATION**

(45) Date of publication of patent specification: **13.03.85**

(51) Int. Cl.⁴: **C 07 C 69/14**, C 07 C 67/36, B 01 J 31/28

(21) Application number: **82830082.2**

(22) Date of filing: **05.04.82**

# (54) Process for producing ethyl acetate by homologation of methyl acetate.

(30) Priority: **06.04.81 IT 2095481**

(43) Date of publication of application:
**20.10.82 Bulletin 82/42**

(45) Publication of the grant of the patent:
**13.03.85 Bulletin 85/11**

(84) Designated Contracting States:
**AT BE CH DE FR GB LI LU NL SE**

(56) References cited:
**EP-A-0 031 606**
**WO-A-81/00856**
**DE-A-2 610 035**
**DE-A-2 733 663**
**GB-A-2 029 409**
**US-A-4 265 828**

**JOURNAL OF THE AMERICAN CHEMICAL SOCIETY Vol. 100, No. 19, 1978 Columbus, Ohio, USA G. BRACA et al. "Simultaneous Carbonylation and Homologation of Dimethyl Ether and Homologation of Methyl Acetate to Ethyl Acetate in the Presence of Ruthenium Catalysts"**

(73) Proprietor: **CONSIGLIO NAZIONALE DELLE RICERCHE**
**Piazzale del Scienze, 7**
**I-00100 Roma (IT)**

(72) Inventor: **Braca, Giuseppe**
**Viale delle Piagge, 8**
**Pisa (IT)**
Inventor: **Sbrana, Glauco**
**Via Rismondo, 31**
**Pisa (IT)**
Inventor: **Valentini, Giorgio**
**Via Friuli, 7**
**Pisa (IT)**

(74) Representative: **Gervasi, Gemma et al**
**Studio Brevetti e Marchi NOTARBARTOLO & GERVASI 33, Viale Bianca Maria**
**I-20122 Milano (IT)**

(56) References cited:
**JOURNAL OF ORGANOMETALLIC CHEMISTRY Vol. 222, No. 1, 15 December 1981 Lausanne J. KEISTER et al. "Promotion by Phosphorus Compounds of Ruthenium-catalyzed Methyl Formate Homologation"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 063 105 B1

Courier Press, Leamington Spa, England.

## Description

This invention relates to a new process for producing ethyl acetate hy homologation of methyl acetate. Ethyl acetate is known as a product widely used in organic chemistry, and is by far the most industrially important acetic ester.

A process is known (USA patent 4,189,441) for producing ethyl acetate from dimethyl ether or methyl acetate by reaction with carbon monoxide and hydrogen. The process is characterized by the use of a catalytic system constituted by a ruthenium carbonyl or an iodised or brominated compound.

This process gives good results when dimethyl ether is used as the substrate, but is much less useful when applied to the conversion of methyl acetate.

In this respect, in such a case in addition to unsatisfactory selectivity in terms of ethyl acetate and products for recycling, there is the simultaneous production of large hydrocarbon quantities, these being undesirable derivatives from the process aspect because they represent low value products. Moreover, at the end of the reaction there is a high concentration of water produced during the homologation, which forms an azeotrope with the ethyl acetate and complicates the normal distillation separation, and in addition gives rise to hydrolytic phenomena on the esters with the formation of methanol and ethanol which can interact with the Ru catalysts to give Ru alkyl intermediates which are easily hydrogenated to hydrocarbons.

The production of high-boiling products (propionic acid and propionates) is also relatively high, with their consequent accumulation in the bottom fraction containing the catalyst, which is recycled.

On the other hand, the considerable development of the BASF and Monsanto processes for the direct carbonylation of methanol, by means of which a mixture of acetic acid and methyl acetate is produced in an extremely convenient manner, makes this latter particularly interesting as a raw material for the production of ethyl acetate.

The object of the present invention is therefore to provide a new process for the homologation of methyl acetate to ethyl acetate, which gives high yields of useful products and high ethyl acetate selectivity. A further object of the present invention is to provide an ethyl acetate preparation process which as its starting material uses a mixture of methyl acetate and acetic acid, in particular as originates from the direct carbonylation of methanol.

The new ethyl acetate preparation process according to the present invention consists essentially of reacting methyl acetate in an acetic acid solution, with carbon monoxide and hydrogen at a temperature of between 150° and 250°C and at a pressure of between 49 and 197 bar, in the presence of a catalyst which can be represented by the formulas:

$$Ru(CO)_xL_yI_2 \qquad (1)$$

$$[Ru(CO)_xL_yI_3]^- \qquad (2)$$

where L is a nitrogenated or phosphorated complexing agent, x is a whole number between 1 and 3, and y is 1 or 2.

The catalyst of formula (1) or (2) which characterises the new process according to the present invention can be added to the reaction system in the form of a preformed complex, or alternatively can be formed directly in the reaction mixture by adding one or more compounds chosen from each of the following classes:

a) carbonyl ruthenium compounds such as $Ru_3(CO)_{12}$, $Ru(CO)_4I_2$, $Na[Ru(CO)_3I_3]$, $[Ru(CO)_3Cl_2]_2$, or ruthenium compounds which under the reaction conditions form ruthenium carbonyls in situ, such as finely divided metal ruthenium, ruthenium tris (acetylacetonate), ruthenium salts of carboxylic acids, sodium or potassium hexachororuthenate, ruthenium halides and the like;

b) iodised compounds chosen from the group consisting of iodine, hydriodic acid, alkyl, aryl or acyl iodides, inorganic iodides and tetralkylammonium iodides;

c) nitrogenated or phosphorated ruthenium complexing agents chosen from the group consisting of nitrogenated heterocyclic or alicyclic bases and phosphines of formula $R_1R_2R_3P$ in which $R_1$, $R_2$, $R_3$, which may be the same or different, are alkyl groups of 1 to 6 carbon atoms, cycloalkyl groups of 3 to 6 carbon atoms, or simple or substituted aryl groups. Examples of nitrogenated bases which are particularly suitable for the purpose of the present invention are pyridine, quinoline, isoquinoline, imidazole, morpholine, piperidine, pyrimidine, pyridazine, thiazole, dipyridyl and benzimidazole, either unsubstituted or substituted.

The compounds pertaining to said classes (a), (b), (c) must be added to the reaction system such that the ruthenium compound:iodised compound:complexing agent molar ratio lies between 1:2:1 and 1:50:50, and preferably between 1:5:5 and 1:10:10.

As stated heretofore, preformed ruthenium iodocarbonyl complexing agents can be added directly to the reaction system, some examples of these being $[RuI_2(CO)_2PPh_3]_2$, $RuI_2(CO)_2(PPh_3)_2$, $[RuI_2(CO)_2PBu_3]_2$, $RuI_2(CO)_2Py_2$, $RuI_2(CO)_3$ (dipy), where Ph is phenyl, Bu is n.butyl, Py is pyridine, and dipy is $\alpha,\alpha'$-dipyridyl.

In all cases, the catalytic complex must be present in a molar proportion of between 1 and 3‰ of the treated methyl acetate.

The reaction according to the present invention must be carried out at a temperature of between 150° and 250°C. The lower temperatures within the defined range can be used if the catalyst comprises a nitrogenated complexing agent, because this type of complexing agent accelerates the reaction. For example, if a catalyst comprising pyridine is used, the highest ethyl acetate selectivity is obtained at 180°—200°C.

If however a catalyst is used comprising a phosphorated complexing agent, it is convenient to work at the higher temperatures, between 200° and 250°C, because these complexing agents reduce the reaction rate.

The $CO/H_2$ mixture fed into the reaction space must ensure a partial pressure of CO which is sufficient to prevent the ruthenium carbonyl derivative decomposing to metal ruthenium. In all cases, the total pressure must be at least 49 bar, and preferably 99—296 Bar.

The $H_2/CO$ ratio in the gaseous reaction mixture can vary between 0.1 and 10. The preferred ratio lies between 0.5 and 2.5. A large hydrogen excess accelerates the reaction, but can limit selectivity by favouring the formation of methane and ethane. A large carbon monoxide excess reduces the reaction rate and favours carbonylation of the methyl acetate to acetic anhydride which, in the presence of the water formed in the homologation, is hydrolysed to acetic acid.

The reaction is preferably carried out in the presence of acetic acid and possibly other recycle products and by-products of the reaction, in which the catalyst is soluble.

As stated heretofore, one of the advantages of the present invention is the fact that it is possible to use, as substrate, the reaction mixture originating directly from the carbonylation of methanol and consisting essentially of methyl acetate and acetic acid.

A further advantage of the new catalytic system derives from its activity in the conversion of water gas:

$$CO+H_2O \rightleftharpoons CO_2+H_2$$

which is displaced towards the production of hydrogen, used in the reaction, as consuming most of the water which forms in the reaction. The elimination of the reaction water is a particularly important aspect, because the ethyl acetate is recovered from the reacted mixture by distillation, and if water is present an azeotrope forms which requires a subsequent purification stage in order to remove the water. Moreover, as stated heretofore, water hydrolyses the methyl and ethyl esters with the production of alcohols which produce hydrocarbons by forming complexes with the Ru catalyst.

In the described process, a gaseous hydrocarbon fraction is generally produced which is eliminated together with small quantities of bottom products constituted by propionic acid and propionates.

When the ethyl acetate has been separated, the intermediate fractions can be all recycled in that they consist of methyl acetate or products (acetic acid, methanol, ethanol, $C_1$ and $C_2$ ethers) which can be all reconverted into methyl acetate or ethyl acetate under the reaction conditions.

In particular, the acetic acid fraction contains the catalyst in solution, and this is also recycled.

Some significant examples are given hereinafter in order to illustrate the process according to the present invention.

Example 1

0.36 mmoles of $Ru(CO)_4I_2$ (prepared by the method described by F. Calderazzo and F. L'Eplattenier, Inorg. Chem., 6, 1220 (1967)), 3.6 mmoles of $CH_3I$ and 3.6 mmoles of pyridine ($Ru:CH_3I:Py=1:10:10$) together with 0.180 moles of methyl acetate (15 ml) and 0.180 moles of acetic acid (10.5 ml) are placed in a Hastelloy C autoclave of 150 ml capacity.

A $CO/H_2$ mixture of molar ratio 1:2 is compressed into the autoclave to a pressure of 148 bar. The autoclave is then placed in a bath temperature-controlled at 200°C and kept stirring for 10 hours, while keeping the pressure constant at $237\pm5$ bar by feeding a 1:1 mixture of $H_2$ and CO.

After the autoclave has cooled, the liquid mixture (25.2 g) and gaseous mixture (22 Nl) representing the products is discharged and analysed by gas chromatography.

The methyl acetate conversion is 41%, with the following product selectivities:

| | |
|---|---|
| ethyl acetate | 76.7% |
| acetic acid | 6.8% |
| methanol | 2.5% |
| ethanol | 2.0% |
| ethers ($C_1$ and $C_2$) | 0.6% |
| propionic acid | 0.7% |
| methane | 10.7% |

In addition, the liquid product fraction contains 1.7% of $H_2O$.

A comparison test was carried out in the same autoclave on a reaction mixture consisting of 0.180 moles of methyl acetate and 0.180 moles of acetic acid.

The operating conditions were identical to those described heretofore.

However, in this case a catalytic system was used which did not comprise the nitrogenated complexing agent, but consisted of 0.36 mmoles of $Ru(CO)_4I_2$ and 3.6 mmoles of $CH_3I$.

The following results were obtained:

methyl acetate conversion 35.6%, product selectivities:

| | |
|---|---|
| ethyl acetate | 65.5% |
| acetic acid | — |
| methanol | 6.4% |
| ethanol | 4.8% |
| ethers ($C_1$ and $C_2$) | 2.6% |
| propionic acid and propionates | 1.1% |
| methane | 19.6% |

The liquid fraction contained 4.1% of $H_2O$.

As can be seen from the given examples, when the complexing agent is absent in the catalyst, there is a substantially lower yield of useful products accompanied by greater production of hydrocarbons and high-boiling products. The quantity of $H_2O$ present in the final mixture is also greater.

All these factors considerably complicate the separation and possible recycling of useful products.

Examples 2—11

Tables 1 and 2 relate to various homologation tests carried out in the same operating manner as described in example 1, but varying the catalytic system.

TABLE 1

| Example | 2* | 3 | 4 | 5 | 6 | 7 |
|---|---|---|---|---|---|---|
| Methyl acetate (mmoles) | 180 | 180 | 180 | 180 | 180 | 180 |
| Acetic acid (mmoles) | 180 | 180 | 180 | 180 | 180 | 180 |
| Ru complex | $Ru(CO)_4I_2$ | $Ru(CO)_4I_2$ | $Ru(CO)_4I_2$ | $Ru(CO)_4I_2$ | $Ru(CO)_4I_2$ | $Ru(CO)_4I_2$ |
| (mg) | 168 | 168 | 168 | 168 | 168 | 168 |
| Iodised compound | $CH_3I$ | $CH_3I$ | $CH_3I$ | NaI | $CH_3I$ | $CH_3I$ |
| (mg) | 511 | 511 | 511 | 540 | 511 | 511 |
| Complexing agent | pyridine | pyridine | pyridine | pyridine | piperidine | morpholine |
| (mg) | 284 | 142 | 711 | 284 | 310 | 160 |
| Ru/iodide/compl. agent | 1/10/10 | 1/10/5 | 1/10/25 | 1/10/10 | 1/10/10 | 1/10/5 |
| $p_{co}$ when cold, bar | 49 | 49 | 49 | 49 | 49 | 49 |
| $p_{H_2}$ when cold, bar | 99 | 99 | 99 | 99 | 99 | 99 |
| Conversion % | | | | | | |
| Methyl acetate | 18 | 16 | 31 | 26.6 | 27 | 15.2 |
| Acetic acid | — | 1.5 | — | — | — | — |

TABLE 1 (continued)

| Example | 2* | 3 | 4 | 5 | 6 | 7 |
|---|---|---|---|---|---|---|
| Products | | | Selectivity % | | | |
| Ethyl acetate | 59.1 | 61.5 | 57.7 | 54 | 66.3 | 52.8 |
| Acetic acid | 24.7 | — | 12.3 | 21.8 | 12.3 | 10.0 |
| Methanol | 5.7 | 8.8 | 0.5 | 0.8 | 1.5 | 3.3 |
| Ethanol | 1.4 | 1.2 | 0.4 | 0.5 | 0.8 | 0.4 |
| Methyl ethyl ether | — | 0.4 | — | — | — | — |
| Propionic acid and propionates | — | — | 0.3 | 5.4 | 0.9 | 0.6 |
| Methane | 9.1 | 28.1 | 28.8 | 17.4 | 18.2 | 32.9 |
| Water (mmoles) | 10.2 | 16 | 3.2 | 6.6 | 7.8 | 7.5 |
| Carbon dioxide (mmoles) | — | 15.7 | 18 | 18 | 18 | 2 |

*reaction temperature: 180°C

TABLE 2

| Example | 8 | 9 | 10 | 11 | 12 |
|---|---|---|---|---|---|
| Methyl acetate (mmoles) | 180 | 180 | 180 | 180 | 180 |
| Acetic acid (mmoles) | 180 | 180 | 180 | 180 | 180 |
| Ru complex | $[RuI_2(CO)_2PPh_3]_2$ | $RuI_2(CO)_2(PPh_3)_2$ | $[RuI_2(CO)_2PBu_3]_2$ | $Ru(CO)_4I_2$ | $Ru(CO)_4I_2$ |
| (mg) | 242 | 337 | 110 | 168 | 168 |
| Iodised compound | $CH_3I$ | $CH_3I$ | $CH_3I$ | $CH_3I$ | $CH_3I$ |
| (mg) | 511 | 511 | 256 | 511 | 511 |
| Complexing agent | | | | $PPh_3$ | $P(CH_3)_2Ph$ |
| (mg) | — | — | — | 71 | 37 |
| Ru/iodide/compl. agent | 1/10/1 | 1/10/2 | 1/10/1 | 1/10/1 | 1/10/1 |
| $p_{co}$ when cold bar, | 53 | 54 | 49 | 54 | 49 |
| $p_{H_2}$ when cold, bar | 103 | 99 | 99 | 103 | 99 |
| Conversion % | | | Selectivity % | | |
| Methyl acetate | 24 | 8 | 4 | 16 | 21 |
| Acetic acid | 12 | 6 | 0 | 12 | 12 |

TABLE 2 (continued)

| Example | 8 | 9 | 10 | 11 | 12 |
|---|---|---|---|---|---|
| | | | Selectivity % | | |
| Products | | | | | |
| Ethyl acetate | 67.5 | 58 | 50 | 70.5 | 69 |
| Methanol | 5.1 | 7 | 19.3 | 6 | 6 |
| Ethanol | 2.3 | 1.2 | — | 2.5 | 2.7 |
| Methyl ethyl ether | 3.2 | 2.5 | — | 3.3 | 2.5 |
| Diethyl ether | 0.5 | 0.4 | — | 0.5 | 0.5 |
| Propionic acid and propionates | 0.4 | 0.5 | — | 0.6 | 0.7 |
| Methane | 21 | 30.4 | 30.7 | 16.6 | 18.6 |
| Water (mmoles) | 24 | 12 | 10 | 24.5 | 27.2 |

Example 12

1.68 g (3.6 mmoles) of $Ru(CO)_4I_2$, 5.11 g (36 mmoles) of $CH_3I$, 2.9 ml (36 mmoles) of pyridine, 133 g (1.8 moles) of methyl acetate and 108 g (1.8 moles) of acetic acid were placed in a 1 litre Hastelloy C reactor fitted with a rotating magnetic stirrer and devices for withdrawing liquid and gaseous samples. A 1:$\frac{1}{2}$ $CO/H_2$ mixture was then compressed into the autoclave to a pressure of 130 bar.

The reactor was then heated to a temperature of 200°C while keeping the pressure constant at 178±5 bar by feeding a 1:1 $CO/H_2$ mixture from a vessel under high pressure.

The reaction was continued for 16 hours, and the variation in the liquid product composition with time was followed by withdrawing and analysing samples at various times.

Figure 1 shows the results of these analyses.

After 16 hours, the reactor was sufficiently cooled, and the liquid products (240 g) and gaseous products (4.57 moles) were discharged and analysed by gas chromatography.

The methyl acetate conversion was 62%, and the various product selectivities were as follows:

| | |
|---|---|
| ethyl acetate | 45.1% |
| acetic acid | 27.3% |
| methanol and ethanol | 0.3% |
| propionic acid | 0.3% |
| propionic esters | 0.7% |
| methane and ethane | 26.3% |

The gaseous products contained 0.15 moles of carbon dioxide, and the water concentration in the liquid products was less than 0.5% by weight.

A test was carried out under the same operating conditions, but using a catalytic system which did not comprise the complexing agent, and consisted of 1.43 g (3.6 mmoles) of $Ru(Ac\ ac)_3$ and 5.11 g (36 mmoles) of $CH_3I$.

The reaction was continued for 14 hours, and the variation in the liquid product composition with time was followed by withdrawing and analysing samples at various times.

Figure 2 shows the results of these analyses.

After 14 hours the reactor was cool, and the liquid products (274 g) and gaseous products (4.17 moles) were discharged and analysed by gas chromatography.

The methyl acetate conversion was 76%.

This high conversion is due to the presence in the reaction mixture of a large water quantity (5.6% of the liquid fraction) which hydrolyses the acetate to methanol. However, the presence of $H_2O$ considerably complicates ethyl acetate separation.

The various product selectivities were as follows:

| | |
|---|---|
| ethyl acetate | 46.3% |
| acetic acid | 11.1% |
| methanol and ethanol | 5.0% |
| $C_1$—$C_2$ ethers | 2.0% |
| propionic acid | 0.5% |
| propionic esters and n-propyl derivatives | 7.2% |
| methane and ethane | 27.9% |

As can be seen, without the complexing agent in the catalyst, there is a total selectivity in terms of ethyl acetate and products for recycling of 64.4% against a selectivity of 72.7% in the corresponding example with pyridine

Moreover, in the absence of pyridine there are larger quantities of high-boiling products, hydrolysis products and hydrocarbons.

**Claims**

1. Process for preparing ethyl acetate from methyl acetate through the reaction with carbon

monoxide and hydrogen at temperature of between 150° and 250°C and at pressure of between 49 and 247 bar, in presence of iodocarbonylruthenium catalytic system, characterized in that the catalyst is a compound of formula

(1) $Ru(CO)_xL_yI_2$

or

(2) $[Ru(CO)_xL_yI_3]^-$

in which L is a nitrogenated or phosphorated Ru complexing agent, x is a whole number between 1 and 3 and y is 1 or 2.

2. A process as claimed in claim 1, wherein the nitrogenated or phosphorated complexing agent is chosen from the group consisting of nitrogenated heterocyclic bases, nitrogenated alicyclic bases, or phosphorated compounds of formula $R_1R_2R_3P$ in which $R_1$, $R_2$, $R_3$, which can be the same or different, are alkyl groups of 1 to 6 carbon atoms, cycloalkyl groups of 3 to 6 carbon atoms, or aryl groups, either simple or substituted.

3. A process as claimed in claim 1, wherein the catalyst is formed directly in the reaction mixture by adding a compound chosen from each of the following classes:

a) carbonyl ruthenium compounds or compounds which form ruthenium carbonyls under the reaction conditions,

b) iodised compounds,

c) nitrogenated or phosphorated Ru complexing agents.

4. A process as claimed in claim 3, wherein the Ru compound of class (a) is chosen from the group consisting of $Ru_3(CO)_{12}$, $Ru(CO)_4I_2$, $Na[Ru(CO)_3I_3]$, $[Ru(CO)_3Cl_2]_2$, finely divided ruthenium metal, Ru salts of carboxylic acids, sodium or potassium hexachlororuthenate, and ruthenium halides.

5. A process as claimed in claim 3, wherein the iodised compound of class (b) is chosen from the group consisting of iodine, hydriodic acid, alkyl iodides, aryl iodides, acyl iodides, inorganic iodides and tetraalkylammonium iodides.

6. A process as claimed in claim 3, wherein the complexing agent of class (c) is chosen from the group consisting of nitrogenated heterocyclic or alicyclic bases and phosphorated compounds of formula $R_1R_2R_3P$, in which $R_1$, $R_2$, $R_3$, which can be the same or different, are alkyl groups of 1 to 6 carbon atoms, cycloalkyl groups of 3 to 6 carbon atoms, or aryl groups, either simple or substituted.

7. A process as claimed in claim 6, wherein the nitrogenated heterocyclic base is chosen from the group consisting of pyridine, quinoline, isoquinoline, imidazole, morpholine, piperidine, pyrimidine, pyridazine, thiazole, dipyridyl, and benzimidazole.

8. A process as claimed in claim 3, wherein the compounds a:b:c are present in the catalytic system in proportions of between 1:2:1 and 1:50:50, and preferably between 1:5:5 and 1:10:10.

9. A process as claimed in claim 1, carried out in the presence of acetic acid.

**Patentansprüche**

1. Verfahren zum Herstellen von Äthylacetat aus Methylacetat durch die Umsetzung von Kohlenmonoxid und Wasserstoff bei einer Temperatur zwischen 150 und 250°C und bei einem Druck zwischen 49 und 247 bar, in Anwesenheit eines katalytischen Jodcarbonylrutheniumsystems, dadurch gekennzeichnet, daß der Katalysator eine Verbindung der Formel

(1) $Ru(CO)_xL_yJ_2$ oder

(2) $[Ru(CO)_xL_yJ_3]^-$

ist, worin L ein nitrogeniertes oder phosphoriertes komplexbildendes Ru-Mittel darstellt, x eine ganze Zahl zwischen 1 und 3 ist und y 1 oder 2 ist.

2. Verfahren, wie in Anspruch 1 beansprucht, worin das nitrogenierte oder phosphorierte komplexbildende Mittel ausgewählt ist aus der Gruppe bestehend aus nitrogenierten heterocyclischen Basen, nitrogenierten alicyclischen Basen oder phosphorierten Verbindungen der Formel $R_1R_2R_3P$, worin $R_1$, $R_2$ und $R_3$, die gleich oder verschieden sein können, Alkylgruppen mit 1 bis 6 C-Atomen, Cycloalkylgruppen mit 3 bis 6 C-Atomen oder Arylgruppen, entweder einfach oder substituiert, sind.

3. Verfahren, wie in Anspruch 1 beansprucht, worin der Katalysator direkt in der Reaktionsmischung durch Zusetzen einer Verbindung ausgewählt aus jeder der folgenden Klassen:

a) Carbonylrutheniumverbindungen oder Verbindungen, die unter den Reaktionsbedingungen Rutheniumcarbonylverbindungen bilden,

b) jodierten Verbindungen, und

c) nitrogenierten oder phosphorierten komplexbildenden Ru-Mitteln gebildet wird.

4. Verfahren, wie in Anspruch 3 beansprucht, worin die Ru-Verbindung von Klasse (a) ausgewählt

wird aus der Gruppe bestehend aus $Ru_3(CO)_{12}$, $Ru(CO)_4J_2$, $Na[Ru(CO)_3J_3]$, $[Ru(CO)_3Cl_2]_2$, fein zerteiltem Rutheniummetall, Ru-Salzen von Carbonsäuren, Natriumoder Kaliumhexachlorruthenat und Rutheniumhalogeniden.

5. Verfahren, wie in Anspruch 3 beansprucht, worin die jodierte Verbindung von Klasse (b) ausgewählt wird aus der Gruppe bestehend aus Jod, Jodwasserstoffsäure, Alkyljodiden, Aryljodiden, Acyljodiden, anorganischen Jodiden und Tetraalkylammoniumjodiden.

6. Verfahren, wie in Anspruch 3 beansprucht, worin das komplexbildende Mittel von Klasse (c) ausgewählt wird aus der Gruppe bestehend aus nitrogenierten heterocyclischen oder alicyclischen Basen und phosphorierten Verbindungen der Formel $R_1R_2R_3P$, worin $R_1$, $R_2$ und $R_3$, die gleich oder verschieden sein können, Alkylgruppen mit 1 bis 6 C-Atomen, Cycloalkylgruppen mit 3 bis 6 C-Atomen oder Arylgruppen, entweder einfach oder substituiert, bedeuten.

7. Verfahren, wie in Anspruch 6 beansprucht, worin die nitrogenierte heterocyclische Base ausgewählt wird aus der Gruppe bestehend aus Pyridin, Chinolin, Isochinolin, Imidazol, Morpholin, Piperidin, Pyrimidin, Pyridazin, Thiazol, Dipyridyl und Benzimidazol.

8. Verfahren, wie in Anspruch 3 beansprucht, worin die Verbindungen a:b:c im katalytischen System in Verhältnissen zwischen 1:2:1 und 1:50:50, vorzugsweise zwischen 1:5:5 und 1:10:10, vorhanden sind.

9. Verfahren, wie in Anspruch 1 beansprucht, durchgeführt in Anwesenheit von Essigsäure.

## Revendications

1. Procédé de préparation d'acétate d'éthyle à partir d'acétate de méthyle par réaction sur de l'oxyde de carbone et de l'hydrogène à une température comprise entre 150 et 250°C et sous une pression comprise entre 49 et 247 bars, en présence d'un système catalytique à base de ruthénium iodocarbonylé, caractérisé en ce que le catalyseur est un composé répondant à la formule

(1) $Ru(CO)_xL_yI_2$ ou

(2) $[Ru(CO)_xL_yI_3]$

où L désigne un agent complexant de Ru azoté ou phosphoré, x est un nombre entier compris entre 1 et 3, et y est égal à 1 ou 2.

2. Procédé selon la revendication 1, caractérisé en ce qu'on choisit l'agent complexant azoté ou phosphoré parmi des bases hétérocycliques azotées, des bases alicycliques azotées ou des composés phosphorés répondant à la formule $R_1R_2R_3P$, où $R_1$, $R_2$ et $R_3$, qui peuvent être identiques ou différents, sont des groupes alcoyle de 1 à 6 atomes de carbone, des groupes cyclo-alcoyle de 3 à 6 atomes de carbone ou des groupes aryle, simples ou substitués.

3. Procédé selon la revendication 1, caractérisé en ce qu'on forme le catalyseur directement dans le mélange réactionnel en ajoutant un composé choisi dans chacune des catégories suivantes:

a) des composés carbonylés du ruthénium ou des composés qui forment des composés carbonylés du ruthénium dans les conditions de réaction,

b) des composés iodés,

c) des agents de complexation de Ru azotés ou phosphorés.

4. Procédé selon la revendication 3, caractérisé en ce qu'on choisit le composé de Ru de la catégorie (a) dans le groupe comprenant $Ru_3(CO)_{12}$, $Ru(CO)_4I_2$, $Na[Ru(CO)_3I_3]$, $[Ru(CO)_3Cl_2]_2$, le ruthénium à l'état de métal finement divisé, des sels de Ru d'acides carboxyliques, de l'hexachlororuthénate de sodium ou de potassium, et des hydrogénures de ruthénium.

5. Procédé selon la revendication 3, caractérisé en ce qu'on choisit le composé de la catégorie (b) dans le groupe comprenant l'iode, l'acide iodhydrique, des iodures d'alcoyle, des iodures d'aryle, des iodures d'acyle, des iodures minéraux et des iodures de tétraalkylammonium.

6. Procédé selon la revendication 3, caractérisé en ce que l'on choisit l'agent complexant de la catègorie (c) dans le groupe comprenant des bases hétérocycliques ou alicycliques azotées et des composés phosphorés répondant à la formule $R_1R_2R_3P$, où $R_1$, $R_2$, $R_3$, qui peuvent être identiques ou différents, sont des groupes alcoyle de 1 à 6 atomes de carbone, des groupes cyclo-alcoyle de 3 à 6 atomes de carbone, ou des groupes aryle, simples ou substitués.

7. Procédé selon la revendication 6, caractérisé en ce qu'on choisit la base hétérocyclique azotée dans le groupe comprenant la pyridine, la quinoleine, l'isoquinoléine, l'imidazole, la morpholine, la pipéridine, la pyrimidine, la pyridazine, le thiazole, le dipyridyle et le benzimidazole.

8. Procédé selon la revendication 3, caractérisé en ce que les composés a:b:c sont présents dans le système catalytique dans des proportions comprises entre 1:2:1 et 1:50:50 et, de préférence, entre 1:5:5 et 1:10:10.

9. Procédé selon la revendication 1, caractérisé en ce qu'on le met en oeuvre en présence d'acide acétique.

FIG 1

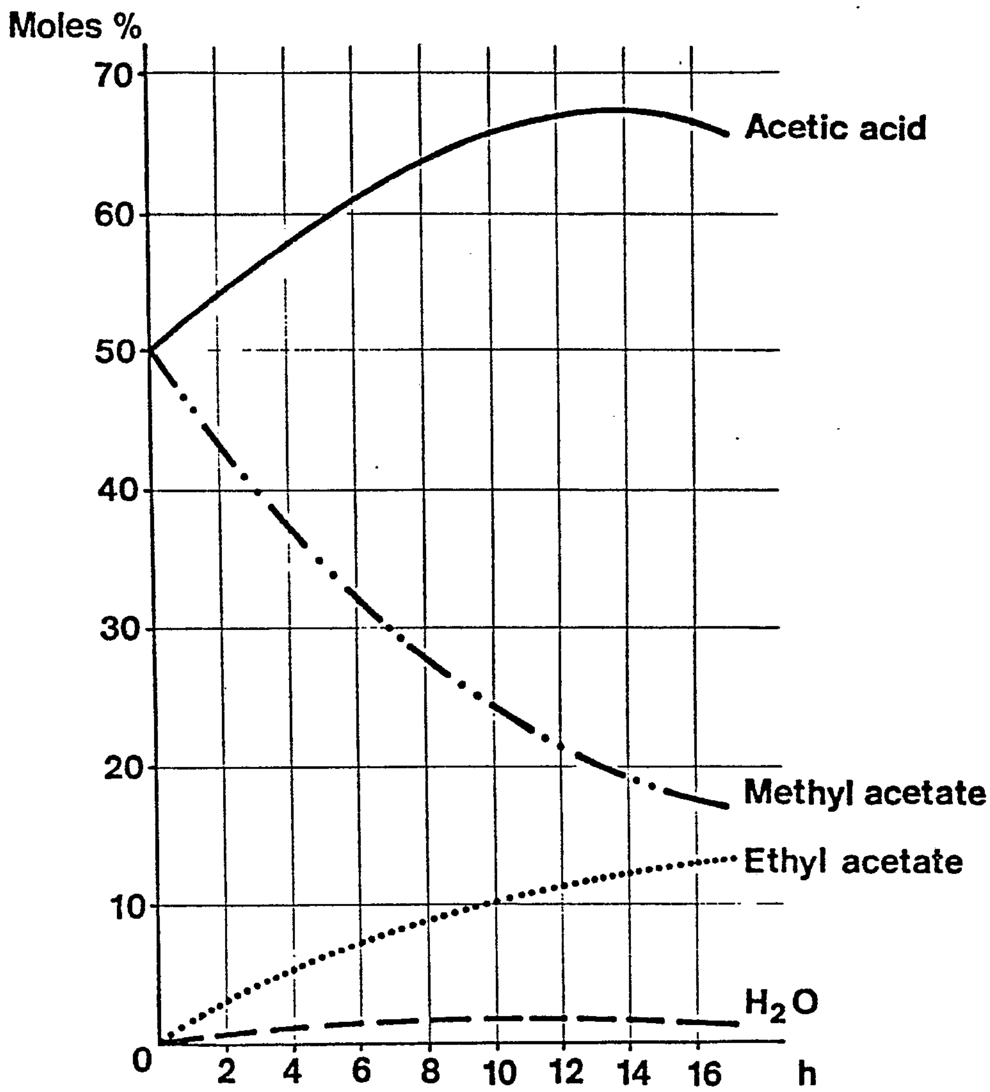
Moles %
70
60
50
40
30
20
10
0
2
4
6
8
10
12
14
16
h
Acetic acid
Methyl acetate
Ethyl acetate
$H_2O$

FIG 2

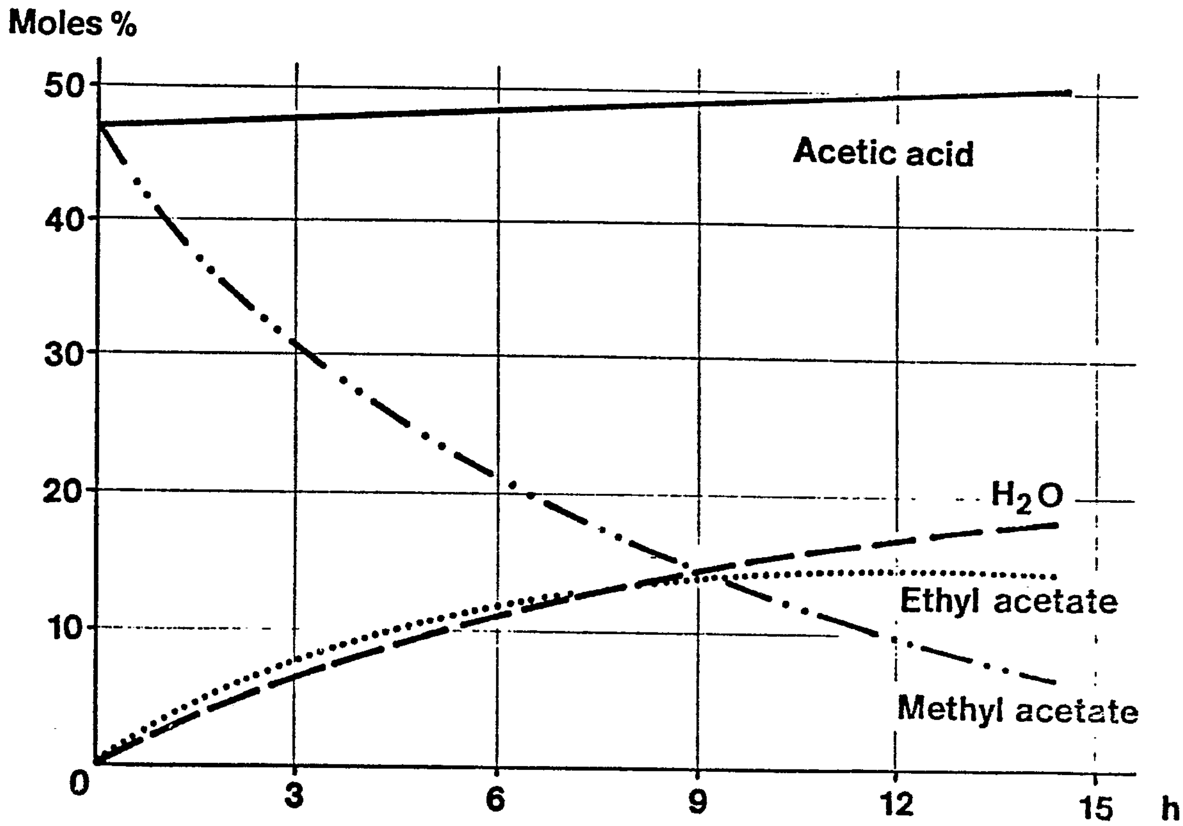
Moles %
50
40
30
20
10
0
3
6
9
12
15
h
Acetic acid
H2O
Ethyl acetate
Methyl acetate